# EUROPEAN PATENT APPLICATION

(11) **EP 2 671 566 A1**
(43) Date of publication of application: **11.12.2013**
(21) Application number: 12742482.8
(22) Date of filing: 02.02.2012
(51) Int. Cl.: A61K 8/67, A61Q 19/08

(54) **EXTERNAL SKIN PREPARATION**

(30) Priority: 02.02.2011 JP 2011020971
(71) Applicant: JX Nippon Oil & Energy Corporation, Chiyoda-ku Tokyo 100-8162 (JP)
(72) Inventor: MAOKA Takashi, Kyoto-shi Kyoto 606-0805 (JP); ISHIBASHI Takashi, Tokyo 100-8162 (JP)
(74) Representative: Dick, Alexander
(86) International application number: PCT/JP2012/052330
(87) International publication number: WO 2012/105632

(57) **Abstract**

This invention provides an external skin preparation with high safety aimed at wrinkle improvement and wrinkle prevention. Specifically, the invention relates to an external skin preparation comprising, as an active ingredient, a carotenoid.

## Description

### Technical Field

The present invention relates to external skin preparations, including cosmetic agents aimed at wrinkle improvement, wrinkle prevention, and other treatments.

### Background Art

Skin aging, such as wrinkles, blotches, a dull complexion, sagging skin, or reduced skin resilience, caused by aging, dry air, sunlight (ultraviolet rays), and other factors, is induced by reduced collagen and elastin in the skin dermis, reduced mucopolysaccharides, cellular damage caused by ultraviolet rays, and the like. In particular, wrinkles that are deep furrows in rhomboid patterns caused by ultraviolet rays are the symptoms of "photoaging."

In the past, photoaging had been treated with the external use of steroids or indomethacin for inhibition of histamine release. However, such pharmaceutical products disadvantageously induce growth and darkening of the hair, atrophic skin conditions, dilated capillaries, or peliosis, as side effects.

Examples of other known treatment techniques include prevention of wrinkle formation with the use of inhibition of histamine release (Patent Document 1), reconstruction of dermal collagen fiber bundles (Patent Document 2), and fructosyl dipeptide or a salt thereof (Patent Document 3).

Astaxanthin, adonirubin, and adonixanthin are carotenoids that are widely distributed among animals, plants, and microorganisms. Astaxanthin is known to have effects of, for example, singlet oxygen quenching, anti-fatigue, anti-inflammation, immunopotentiation, endurance-strengthening, melanin production control, and wrinkle formation suppression (Non-Patent Document 1). In addition, wrinkle formation caused by photoaging is known to be prevented with the external use of an antioxidant, an anti-inflammatory agent, a chelating agent, or the like (Non-Patent Documents 2 and 3). One of the reasons for wrinkle formation caused by photoaging is cross-linking of dermal collagen by singlet oxygen. Astaxanthin is known to prevent dermal collagen from being crosslinked by singlet oxygen with its singlet oxygen quenching capacity and to prevent wrinkle formation caused by photoaging (Non-Patent Document 1).

However, it was not known in the past that adonirubin and adonixanthin had singlet oxygen quenching capacity, which is important for prevention of photoaging, or that such capacity was higher than that of astaxanthin.

### Prior Art Documents

### Patent Documents

Patent Document 1: JP Patent Publication (Kokai) No. 2002-241300 A
Patent Document 2: JP Patent Publication (Kokai) No. 2002-255781 A
Patent Document 3: JP Patent Publication (Kokai) No. 2010-180240 A

### Non-Patent Documents

Non-Patent Document 1: Kazuyoshi Yazawa, "Astaxanthin no Kagaku" ("Sciences of Astaxanthin"), Sezando-Shoten Publishing Co., Ltd., November, 2009
Non-Patent Document 2: Bissett, D. L. et al., "Photodermatol. Photoimmunol. Photomed.," 1990, vol. 7, pp. 56-62
Non-Patent Document 3: Bissett, D. L. et al., "J. Soc. Cosmet. Chem.," 1992, vol. 43, pp. 85-92

### Summary of the Invention

### Object to Be Attained by the Invention

Under the above circumstances, it is an object of the present invention to provide an external skin preparation with high safety having singlet oxygen quenching capacity.

### Means for Attaining the Object

The present inventors have conducted concentrated studies in order to attain the above object. As a result, they have found that adonirubin and adonixanthin have higher singlet oxygen quenching capacity than astaxanthin. This has led to the completion of the present invention.

The present invention includes the following.
(1) An external skin preparation comprising, as an active ingredient, a carotenoid.
(2) The external skin preparation according to (1), wherein the carotenoid is adonirubin and/or adonixanthin.
(3) The external skin preparation according to (1) or (2), wherein the carotenoid is adonirubin.
(4) The external skin preparation according to (2) or (3), wherein the adonirubin is in a free form.
(5) The external skin preparation according to (1) or (2), wherein the carotenoid is adonixanthin.
(6) The external skin preparation according to (2) or (5), wherein the adonixanthin is in a free form.
(7) The external skin preparation according to (1), wherein the carotenoid is a mixture containing adonirubin, adonixanthin, and astaxanthin.
(8) The external skin preparation according to any one of (1) to (7), wherein the carotenoid is derived from *Paracoccus carotinflaciens.*
(9) The external skin preparation according to any one of (1) to (8) for wrinkle improvement and/or wrinkle prevention.
(10) A cosmetic agent comprising the external skin preparation according to any one of (1) to (9).

This description includes part or all of the content as disclosed in the description and/or drawings of Japanese Patent Application No. 2011-020971, which is a priority document of the present application.

### Effects of the Invention

The present invention provides a carotenoid-containing external skin preparation with high safety.

### Brief Description of the Drawing

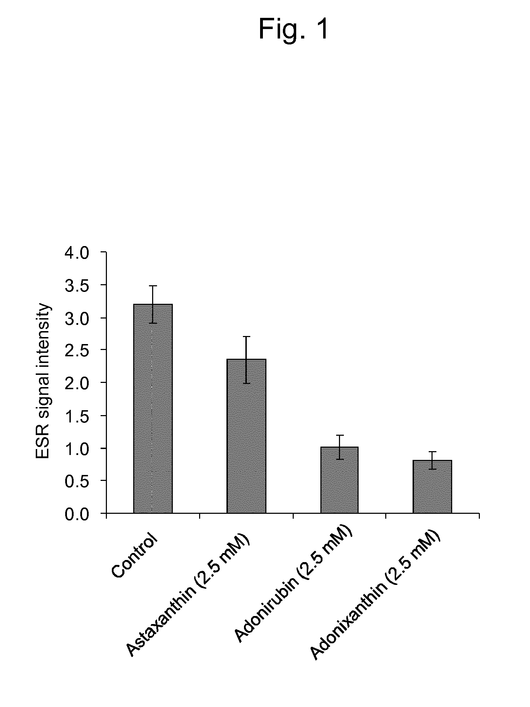

Fig. 1 shows a chart demonstrating the singlet oxygen quenching capacity of carotenoids (i.e., adonirubin, adonixanthin, and astaxanthin) measured in Example 1.

### Embodiments for Carrying out the Invention

Hereafter, the present invention is described in detail.

The external skin preparation according to the present invention comprises, as an active ingredient, a carotenoid. The external use of the external skin preparation according to the present invention for humans enables improvement and prevention of wrinkles caused by photoaging without side effects.

The present invention involves the use of a carotenoid as an active ingredient. Examples of carotenoids include adonirubin, adonixanthin, and a mixture of adonirubin and adonixanthin. Use of a carotenoid in a free form is also preferable.

Commercially available carotenoids may be used. Alternatively, carotenoids produced via conventional techniques, such as chemical synthesis, microorganism-induced fermentation, or extraction and purification from animals or plants (i.e., naturally-occurring carotenoids), can be used. For example, dried cells of *Paracoccus carotinifaciens* are subjected to extraction at room temperature with the use of acetone, and the extract is concentrated with an evaporator. When the concentrate is separated into two phases, a hexane/chloroform (1:1) mixture is added thereto, the resultant is thoroughly mixed, and an organic solvent phase is then obtained via separation. The organic solvent phase is concentrated to dryness using an evaporator. The dry concentrate is dissolved in chloroform, and carotenoids are separated with the use of silica gel columns. For example, a fraction eluted with 300 ml of acetone/hexane (3:7) is further purified via HPLC (Shim-pack PRC-SIL, acetone/hexane (3:7)) to obtain adonirubin in a free form. Alternatively, a fraction eluted with acetone/hexane (5:5) is concentrated, and the concentrate is allowed to stand at 4°C. Thus, astaxanthin in a free form can be obtained as a crystal. Further, a fraction eluted with acetone may be purified via HPLC (Shim-pack PRC-SIL, acetone/hexane (4:6)) to obtain adonixanthin in a free form.

A carotenoid mixture containing astaxanthin (Non-Patent Document 1), which is known to have singlet oxygen quenching capacity, in addition to adonirubin and adonixanthin, can also be used as the carotenoid. For example, a carotenoid mixture extracted from dried cells of *Paracoccus carotinifaciens* in accordance with the methods described in JP Patent Publication (Kokai) No. 2007-261972 A and JP Patent Publication (Kokai) No. 2009-50237 A contains carotenoids (i.e., astaxanthin, adonirubin, and adonixanthin) in the composition percentages shown in Tables 1 and 2 below. Table 1 shows the composition percentages for the extracted carotenoid mixture. The carotenoid composition percentages can be freely adjusted in accordance with culture conditions for *Paracoccus carotinflaciens.* Table 2 shows the carotenoid composition percentages per dry cell of *Paracoccus carotinflaciens.*

**Table 1**

| Carotenoid types | Composition percentage (wt %) |
|---|---|
| Astaxanthin | 30 to 60 |
| Adonirubin | 15 to 50 |
| Adonixanthin | 10 to 50 |
| Other carotenoid | 5 to 20 |

**Table 2**

| Carotenoid types | Composition percentage (wt %) |
|---|---|
| Astaxanthin | 0.3 to 2.4 |
| Adonirubin | 0.15 to 2.0 |
| Adonixanthin | 0.1 to 2.0 |
| Other components* | 96 to 99 |

| | |
|---|---|
| * Containing other carotenoids, bacteria-derived carbohydrates, or the like. | |

Accordingly, a substance extracted from the dried cells of *Paracoccus carotinifaciens* can be used as a carotenoid mixture containing adonirubin, adonixanthin, and astaxanthin.

The external skin preparation according to the present invention can be produced with the use of the above-described carotenoids as active ingredients.

The external skin preparation according to the present invention can be used in the form of, for example, a lotion, an emulsion, cleansing cream, nourishing cream, pre-makeup cream, foundation, body lotion, hand cream, leg cream, face wash, or body wash. The state thereof is not particularly limited, and it can be used in the state of, for example, a lotion, liquid, cream, gel, emulsion, or solid.

In addition to carotenoids, the external skin preparation according to the present invention can contain other components, according to need. Examples of such components include: hydrocarbons, such as liquid paraffin and vaseline; waxes, such as carnauba wax and Japan wax; oils and fats, such as olive oil and jojoba oil; esters, such as octadecyl palmitate and neopentyl glycol diisooctanoate; higher fatty acids, such as stearic acid and palmitic acid; higher alcohols, such as cetyl alcohol and stearyl alcohol; nonionic, anionic, cationic, or amphoteric surfactants; natural or synthetic aromatics and pigments; parabens; higher alcohols, such as chlorhexidine gluconate; pH modifiers, such as citrate and acetate; and substances with various medicinal properties suitable for the purposes of use. In addition, various ingredients of external skin preparations, such as general cosmetic and pharmaceutical products, can be adequately incorporated. The external skin preparation according to the present invention can also be applied to a patient in combination with another external skin preparation or in the form of a mixture thereof with another external skin preparation.

The amount of carotenoids contained in the external skin preparation according to the present invention varies depending on various factors, such as the age, body weight, sexuality, or conditions of the patient, or severity of the affliction. For example, carotenoid is incorporated in an amount of 0.00001 % to 5 % by weight, and preferably 0.0001 % to 1% by weight, relative to the total weight of the external preparation.

Crosslinking of dermal collagen by singlet oxygen is one reason for wrinkle formation caused by photoaging (Non-Patent Document 1). If the external skin preparation according to the present invention can quench singlet oxygen, specifically, changes in collagen as observed on the photoaged skin can be inhibited, and anti-aging effects can be exerted. Accordingly, pharmacological evaluation of the external skin preparation according to the present invention in respect of improvement and prevention of wrinkle formation can be performed using the singlet oxygen quenching capacity as the indicator. For example, pharmacological evaluation of the external skin preparation according to the present invention can be performed by determining whether or not the external skin preparation according to the present invention has sufficient singlet oxygen quenching capacity by the electron spin resonance (ESR) method that is employed for evaluation of the singlet oxygen quenching activity. According to the ESR method, a lower ESR signal intensity indicates more effective quenching of singlet oxygen.

The external skin preparation according to the present invention described above can be used as a cosmetic agent or pharmaceutical product for wrinkle improvement, prevention, or other purposes without side effects.

In association with the external skin preparation according to the present invention, the present invention relates to the use of a carotenoid in manufacture of a pharmaceutical composition for wrinkle improvement and/or prevention (e.g., an external skin preparation). The present invention also relates to a carotenoid for use in wrinkle improvement and/or prevention.

In association with the external skin preparation according to the present invention, the present invention relates to a method of wrinkle improvement and/or prevention comprising external use (application) of a carotenoid or a pharmaceutical composition comprising, as an active ingredient, a carotenoid (e.g., an external skin preparation) to the skin of a human or non-human animal, and use of a carotenoid or a pharmaceutical composition comprising, as an active ingredient, a carotenoid (e.g., an external skin preparation) in wrinkle improvement and/or prevention.

### Examples

Hereafter, the present invention is described in greater detail with reference to the examples, although the technical scope of the present invention is not limited to these examples.

### [Preparation Example 1] Preparation of adonirubin, adonixanthin, and astaxanthin in a free form

Dried cells of *Paracoccus carotinifaciens* were subjected to extraction at room temperature using acetone. The extract was concentrated with an evaporator. When the concentrate was separated into two phases, a hexane/chloroform (1:1) mixture was added thereto, the resultant was thoroughly mixed, and an organic solvent phase was then obtained via separation.

The organic solvent phase was concentrated to dryness using an evaporator. The dry concentrate was dissolved in chloroform, and carotenoids were separated with the use of silica gel columns. Specifically, a fraction eluted with 300 ml of acetone/hexane (3:7) was further purified via HPLC (Shim-pack PRC-SIL, acetone/hexane (3:7)) to obtain adonirubin in a free form. A fraction eluted with acetone/hexane (5:5) was concentrated, and the concentrate was allowed to stand at 4°C. Thus, astaxanthin in a free form was obtained as a crystal. A fraction eluted with acetone was further purified via HPLC (Shim-pack PRC-SIL, acetone/hexane (4:6)) to obtain adonixanthin in a free form.

In the example below, the products obtained above were used as adonirubin, adonixanthin, and astaxanthin in free forms.

### [Example 1] Evaluation of singlet oxygen quenching activity of carotenoids

The singlet oxygen quenching activity of carotenoids (i.e., adonirubin, adonixanthin, and astaxanthin in free forms) was evaluated in the manner described below.

### 1-1. Experimentation method

Evaluation was carried out by the ESR method using an ESR apparatus (JEOLJES-FR30, JEOL Ltd.). The method of evaluation is described below.

50 µl of 250 mM spin trap agent, 50 µl of an aqueous solution of 2,2,6,6-tetramethyl-4-piperidione (TMPD), and 50 µl of an acetone solution of 10 mM carotenoid were added to 50 µl of 100 mM phosphate buffer (pH 7.4), these components were thoroughly mixed, 50 µl of an aqueous solution of 0.25 mM hematoporphyrin was added thereto as a singlet oxygen generator, and the resultant was irradiated with ultraviolet A (UVA) to generate singlet oxygen.

Subsequently, ESR signals based on stable nitroxyl radicals (4-oxo-TEMPO) generated upon the reaction between singlet oxygen and TMPD in the reaction solution were measured. A lower ESR signal intensity indicates more effective quenching of singlet oxygen.

### 1-2. Experimentation results

The results are shown in Table 3 and Fig. 1. No antioxidant was applied to a control group. The results are the mean and the standard deviations of three independent experiments.

**Table 3**

| | ESR signal intensity | | | Mean | S.D. |
|---|---|---|---|---|---|
| Control | 3.02 | 3.53 | 3.05 | 3.20 | 0.29 |
| Astaxanthin (2.5 mM) | 2.00 | 2.33 | 2.72 | 2.35 | 0.36 |
| Adonirubin (2.5 mM) | 0.79 | 1.13 | 1.11 | 1.01 | 0.19 |
| Adonixanthin (2.5 mM) | 0.74 | 0.97 | 0.72 | 0.81 | 0.14 |

A shown in Table 3 and Fig. 1, the singlet oxygen quenching activities of adonirubin and adonixanthin were each significantly higher than that of astaxanthin.

All publications, patents, and patent applications cited herein are incorporated herein by reference in their entirety.

## Claims

1. An external skin preparation comprising, as an active ingredient, a carotenoid.

2. The external skin preparation according to claim 1, wherein the carotenoid is adonirubin and/or adonixanthin.

3. The external skin preparation according to claim 1 or 2, wherein the carotenoid is adonirubin.

4. The external skin preparation according to claim 2 or 3, wherein the adonirubin is in a free form.

5. The external skin preparation according to claim 1 or 2, wherein the carotenoid is adonixanthin.

6. The external skin preparation according to claim 2 or 5, wherein the adonixanthin is in a free form.

7. The external skin preparation according to claim 1, wherein the carotenoid is a mixture containing adonirubin, adonixanthin, and astaxanthin.

8. The external skin preparation according to any one of claims 1 to 7, wherein the carotenoid is derived from *Paracoccus carotinflaciens.*

9. The external skin preparation according to any one of claims 1 to 8 for wrinkle improvement and/or wrinkle prevention.

10. A cosmetic agent comprising the external skin preparation according to any one of claims 1 to 9.
